Europäisches Patentamt

European Patent Office

Office européen des brevets

(11) Numéro de publication: **0 465 313 B1**

# FASCICULE DE BREVET EUROPEEN

(45) Date de publication de fascicule du brevet: **19.10.94**

(21) Numéro de dépôt: **91401755.3**

(22) Date de dépôt: **27.06.91**

(51) Int. Cl.⁵: **A61K 35/78**, A61K 31/215, A61K 31/22, A61K 7/48, //(A61K35/78,31:215,31:22)

(54) **Composition cicatrisante à usage local.**

(30) Priorité: **29.06.90 FR 9008238**

(43) Date de publication de la demande: **08.01.92 Bulletin 92/02**

(45) Mention de la délivrance du brevet: **19.10.94 Bulletin 94/42**

(84) Etats contractants désignés: **AT BE CH DE DK ES FR GB GR IT LI LU NL SE**

(56) Documents cités:
**FR-A- 2 591 104**
**US-A- 3 920 835**
**US-A- 4 105 783**

(73) Titulaire: **LABORATOIRE D'EVOLUTION DERMATOLOGIOUE- LED**
**Les Espaces Delta,**
**Route du Parc**
**Sophia Antipolis, (Alpes Maritimes) (FR)**

(72) Inventeur: **Allart, Jean-Claude**
**12 rue Lamendin**
**Bruay Labuissière, (Pas de Calais) (FR)**

(74) Mandataire: **Cabinet Pierre HERRBURGER**
**115, Boulevard Haussmann**
**F-75008 Paris (FR)**

**Description**

La présente invention concerne une composition cicatrisante à usage local, plus spécialement destinée à restaurer la couche cornée dans des états déficitaires de kératinisation.

La cicatrisation des plaies consécutives à des traumatismes ou à des affections diverses s'effectue, le plus souvent, d'elle-même, en un temps plus ou moins long, ou bien avec l'aide de médications dont l'efficacité ne s'avère malheureusement pas toujours suffisante.

Parmi les compositions cicatrisantes ayant permis d'obtenir les résultats les plus satisfaisants, on peut noter celle décrite dans le brevet FR 85 18 088 ; celle-ci contient au moins une huile naturelle peroxydée par saturation en oxygène et exposition intensive et contrôlée aux ultraviolets de façon à présenter un taux de peroxydation compris entre 30 et 300, exprimé en milli-équivalents, et une teneur en glycérides oxydés comprise entre 5 et 40, la définition de cette huile en spectrophotométrie UV étant de 1 à 6 pour le facteur E 270, et de 8 à 60 pour le facteur E 232.

Cette composition, qui a en fait une double activité antalgique et cicatrisante, s'est révélée particulièrement efficace pour le traitement de pathologies particulières telles que par exemple l'escarre des malades alités, la fissure anale ou l'ulcère variqueux de la jambe.

Malgré ces avantages, l'utilisation de cette composition n'a, cependant, donné que des résultats insuffisants dans le cas du traitement d'affections particulièrement rebelles telles que, par exemple, les états de déficits de la kératinisation au niveau du tégument et des muqueuses, notamment dans les chéilites, pulpites, eczématides (dartres du visage), épidermolyses, rhinites sèches.

Or, il serait particulièrement avantageux de pouvoir disposer d'une composition efficace vis-à-vis de ces affections : en effet, il est couramment admis, aujourd'hui, qu'un certain nombre de médications à visée dermatologique intégrant des dérivés de la vitamine A, encore dénommés rétinoïdes (isotrétinoïne, étrétinate, acitrétine en particulier), sont responsables de désordres de la kératinisation à type de chéilites (lèvres), de pulpites (extrémités des doigts), de sécheresses cutanéo-muqueuses (nez, conjonctive) considérées comme des effets secondaires gênants. Toutefois, compte tenu de l'activité indéniable de ces composés dans l'affection considérée (acné, psoriasis en particulier) les patients acceptent de supporter ces problèmes locaux.

La présente invention a pour objet de remédier à ces inconvénients en proposant une composition cicatrisante à usage local, plus spécialement destinée à restaurer la couche cornée dans des états déficitaires de kératinisation.

Selon l'invention, cette composition, de type cosmétique pharmaceutique ou plus particulièrement dermo-pharmaceutique, est caractérisée en ce qu'elle contient en tant que principe actif, la combinaison d'au moins une huile naturelle peroxydée et d'acide $\alpha$-acétylmandélique.

On s'est, en effet, aperçu que, de manière surprenante, l'acide $\alpha$-acétylmandélique provoque, là où il est appliqué, une action antagoniste de celle des rétinoïdes (ceux-ci étant appliqués par voie orale), et ce, en entraînant une augmentation de la cohésion cornéocytaire ; il y a synergie entre cette action et celle, propre, des huiles naturelles peroxydées qui correspond à une amélioration de la vascularisation sous-cutanée.

Cette synergie d'action a été mise en évidence, sur le plan clinique, dans le traitement des chéilites de stade III et IV induites par l'administration d'isotrétinoïne peros dans l'acné nodulokystique.

Selon l'invention, la ou les huiles peroxydées sont, de préférence, choisies dans le groupe formé par l'huile d'amande douce peroxydée, l'huile de maïs peroxydée et l'huile de sésame peroxydée.

Bien entendu, cette caractéristique ne doit être aucunement considérée comme limitative de l'invention et on pourrait fabriquer des compositions cicatrisantes à base d'huile animale peroxydée.

Selon une autre caractéristique de l'invention, la composition cicatrisante contient, en tant que principe actif entre 65 et 99 % d'huile peroxydée et entre 1 et 35 % d'acide $\alpha$-acétylmandélique.

Les différents essais effectués ont, en effet, permis de montrer que seule cette association, dans les proportions susmentionnées, était susceptible de permettre d'aboutir aux propriétés recherchées.

A titre d'exemple, le principe actif de la composition cicatrisante objet de l'invention peut correspondre aux combinaisons suivantes :

| Exemple A : | |
|---|---|
| Acide $\alpha$-acétylmandélique | 20 g |
| Huile d'amande douce peroxydée | 80 g |

| Exemple B : | |
|---|---|
| Acide α-acétylmandélique | 25 g |
| Huile de maïs peroxydée | 75 g |

| Exemple C : | |
|---|---|
| Acide α-acétylmandélique | 3 g |
| Huile d'amande douce peroxydée | 97 g |

| Exemple D : | |
|---|---|
| Acide α-acétylmandélique | 10 g |
| Huile de sésame peroxydée | 90 g. |

Selon l'invention, la composition cicatrisante peut se présenter sous diverses formes galéniques, notamment de lait, de crème ou de gel : on peut, en particulier, citer sans que cette énumération soit limitative, les crèmes de soin pour le visage, les crèmes pour les mains, les laits corporels, les laits ou crèmes démaquillants, les gels huileux ainsi que les crèmes et sticks pour les lèvres.

Les exemples ci-dessous citent six compositions cicatrisantes correspondant à l'invention :

| Exemple 1 : Composition d'une crème pour le visage sous forme d'une émulsion eau dans l'huile : | |
|---|---|
| Acide stéarylique | 1,00 g |
| Arginine | 1,50 g |
| Huile de paraffine | 30,00 g |
| Composition cicatrisante selon l'exemple D | 40,00 g |
| Conservateur | 0,15 g |
| Borate de sodium | 10,10 g |
| Parfum | 0,10 g |
| Eau q.s.p. | 100,00 g |

**Exemple 2** : Composition d'un lait corporel (émulsion huile dans l'eau) :

| | |
|---|---|
| Palmito stéarate de propylène glycol | 3,00 g |
| Ether d'alcool gras et de polyéthylène glycol | 3,00 g |
| Huile de vaseline | 15,00 g |
| Composition cicatrisante selon l'exemple A | 30,00 g |
| Conservateur | 0,15 g |
| Parfum | 0,10 g |
| Borate de sodium | 1,00 g |
| Eau q.s.p. | 100,00 g |

| Exemple 3 : Composition d'une crème pour les mains: | |
|---|---|
| Huile de silicone | 3,00 g |
| Huile de vaseline | 10,00 g |
| Glycérine | 10,00 g |
| Composition cicatrisante selon l'exemple A | 20,00 g |
| Conservateur | 0,10 g |
| Parfum | 0,10 g |
| Borate de sodium | 1,00 g |
| Eau q.s.p. | 100,00 g |

## Exemple 4 : Composition d'un lait démaquillant :

| | |
|---|---|
| Huile de vaseline | 5,00 g |
| Ester de sorbitanne | 2,00 g |
| Ester de sorbitanne polyoxyéthyléné | 25,00 g |
| Composition cicatrisante selon l'exemple B | 25,00 g |
| Borate de sodium | 1,00 g |
| Conservateur | 0,10 g |
| Eau q.s.p. | 100,00 g |

| Exemple 5 : Composition d'un gel huileux: | |
|---|---|
| Silice micronisée | 7,00 g |
| Composition cicatrisante selon l'exemple C | 93,00 g |

| Exemple 6 : Composition d'une crème pour les lèvres: | |
|---|---|
| Palmitate de cetyle | 11,75 g |
| Cire d'abeille | 13,50 g |
| Composition cicatrisante selon l'exemple D | 25,00 g |
| Butylhydroxyanisole | 0,01 g |
| Huile de vaseline | 43,30 g |
| Eau q.s.p. | 100,00 g |

Il est à noter que l'invention s'applique de façon particulièrement satisfaisante au traitement des lèvres : l'efficacité toute particulière d'une préparation pour les lèvres sera mise en lumière grâce à l'exemple ci-dessous qui résume les résultats obtenus chez 6 patients ayant reçu une telle préparation :

F.F.R., 16 ans a constaté dès la première heure de l'application de la préparation un effet réparateur qui a perduré pendant les cinq jours d'application.

C.OU., 32 ans, a constaté que dans le même délai (moins d'une heure) le mouvement des lèvres était moins douloureux (chéilites stade III).

DF. RT., 15 ans, a estimé que l'application sur le lieu de consultation avait un effet bénéfique immédiat et que le produit testé était plus efficace à terme que les sticks pour lèvres habituellement prescrits par son dermatologiste.

4

T. FA., souffrant d'une chéilite fissuraire (stade IV) a estime qu'un traitement de deux jours avec application bi-quotidienne avait permis une résolution partielle de la fissure centrale.

F. TH., 18 ans, a noté un délai minimal de deux heures après application de la préparation pour l'obtention d'un effet apaisant au niveau des lèvres.

B.O.N., 16 ans, a constaté vingt quatre heures après la première application les premiers effets bénéfiques sur le dessèchement labial.

Tous ces patients avaient bien sûr maintenu leur pris d'isotrétinoïne pendant l'application de la préparation pour lèvres (cinq jours).

Il ressort à la suite de ces quelques résultats que l'effet bénéfique sur la muqueuse labiale induit par l'application de la préparation conforme à l'invention se manifeste rapidement (délai souvent inférieur à deux heures) et durablement puisqu'après cinq jours de traitement bi-quotidien, aucune récidive de desquamation labiale n'est à noter. La tolérance était jugée excellente par l'ensemble des patients ; il faut signaler à ce sujet que la préparation étudiée ne nécessite par l'adjonction de conservateur et de parfum, composés qui peuvent générer des problèmes de nature allergique.

## Revendications

1. Composition cicatrisante à usage local plus spécialement destinée à restaurer la couche cornée dans des états déficitaires de kératinisation, caractérisée en ce qu'elle contient en tant que principe actif, la combinaison d'au moins une huile naturelle peroxydée et d'acide $\alpha$-acétylmandélique.

2. Composition cicatrisante selon la revendication 1, caractérisée en ce que la ou les huiles peroxydées sont de préférence constituées par des huiles végétales peroxydées notamment choisies dans le groupe formé par l'huile d'amande douce peroxydée, l'huile de maïs peroxydée et l'huile de sésame peroxydée.

3. Composition cicatrisante selon l'une quelconque des revendications 1 et 2, caractérisée en ce qu'elle contient, en tant que principe actif entre 65 et 99 % d'huile peroxydée et entre 1 et 35 % d'acide $\alpha$-acétylmandélique.

4. Composition cicatrisante selon l'une quelconque des revendications 1 à 3, caractérisée en ce qu'elle se présente sous la forme d'un lait, d'une crème ou d'un gel.

5. Composition cicatrisante selon l'une quelconque des revendications 1 à 4, caractérisée en ce qu'elle est constituée par une crème ou un stick pour les lèvres.

## Claims

1. Cicatrising composition for local use, more especially intended for restoring the corneous layer in keratin-deficient states, characterised in that it contains as active component a combination of at least one peroxidised natural oil and $\alpha$-acetylmandelic acid.

2. Cicatrising composition according to Claim 1, characterised in that the peroxidised oil or oils is/are preferably constituted peroxidised vegetable oils in particular selected from the group consisting of peroxidised sweet almond oil, peroxidised corn oil and peroxidised sesame oil.

3. Cicatrising composition according to either of Claims 1 and 2, characterised in that it contains as active component between 65 and 99 % of peroxidised oil and between 1 and 35 % of $\alpha$-acetylmandelic acid.

4. Cicatrising composition according to any one of Claims 1 to 3, characterised in that it is in the form of a milk, a cream or a gel.

5. Cicatrising composition according to any one of Claims 1 to 4, characterised in that it is formed by a cream or a stick for the lips.

**Patentansprüche**

1.  Narbenbildendes Mittel zur örtlichen Anwendung speziell zur Wiederherstellung der Hornschicht bei Schwierigkeiten bei der Hornschichtbildung, gekennzeichnet dadurch, daß dieses als Wirkstoff eine Mischung von mindestens einem natürlichen peroxydierten Öl und einer $\alpha$-Azethylmandelsäure enthält.

2.  Narbenbildendes Mittel nach Anspruch 1, gekennzeichnet dadurch, daß das oder die peroxydierten Öle vorzugsweise pflanzliche peroxydierte Ole sind, insbesondere peroxydierte Süßmandelöle, peroxydierte Maisöle und peroxydierte Sesamöle.

3.  Narbenbildendes Mittel nach einem der Ansprüche 1 und 2, gekennzeichnet dadurch, daß dieses als Wirkstoff 65 bis 99 % peroxydiertes Öl und 1 bis 35 % $\alpha$-Azethylmandelsäure enthält.

4.  Narbenbildendes Mittel nach einem der Ansprüche 1 bis 3, gekennzeichnet dadurch, daß es als Milch, Creme oder Gel auftritt.

5.  Narbenbildendes Mittel nach einem der Ansprüche 1 bis 4, gekennzeichnet dadurch, daß dieses eine Lippencreme oder ein Lippenstift ist.